# EUROPEAN PATENT APPLICATION

(11) **EP 1 752 131 A1**
(43) Date of publication of application: **14.02.2007**
(21) Application number: 05017531.4
(22) Date of filing: 11.08.2005
(51) Int. Cl.: A61K 8/49, A61K 31/353, A61Q 19/00, A61Q 19/08, A61Q 17/00

(54) **Stabilized pharmaceutical and cosmetic composition of catechins or derivatives thereof**

(71) Applicant: Anagen Therapeutics, Inc., Chicago IL 60616 (US)
(72) Inventor: Liao, Shu-Tsung, Chicago Illinois 60616 (US); Liang, Teh-Ming, Chicago Illinois 60616 (US); Liang, Dean, Chicago Illinois 60616 (US)
(74) Representative: Eder, Thomas

(57) **Abstract**

A stabilized pharmaceutical and cosmetic composition of catechins is disclosed. The pharmaceutical composition includes antioxidants, metal chelating agents, catechins or derivatives, and water. The pharmaceutical composition disclosed here can inhibit the oxidation of the catechins and derivatives thereof, and therefore extend the shelf life of the pharmaceutical composition of catechins and increase the medical application of the compostions.

## Description

### BACKGROUND OF THE INVENTION

### FIELD OF THE INVENTION

The present invention generally relates to pharmaceutical composition and, more particularly, to a stabilized pharmaceutical and cosmetic composition of catechins or derivatives thereof.

### DESCRIPTION OF THE RELATED ART

Catechins and the derivatives thereof are known and useful components contained in the extract of leaves of green tea. Nowadays, catechins and their derivatives are used for many medical applications. For example, green tea catechins have been shown to prevent several type of cancer including skin cancer. Studies show that green tea catechins inhibit tumor formation at both the initiation and the promotion stages. Green tea administered in drinking water to mice inhibits skin tumor formation induce by UV irradiation (Wang et al, 1991). EGCG topically applied to skin inhibits teleocidin-induced tumor promotion in mice previously initiated with DMBA (Yoshizawa et al., 1987).

On the other hand, catechins are also antioxidants. The mechanism by which catechins inhibit tumor initiation and promotion may be in part due to their antioxidant activity. UV irradiation and chemical carcinogens produce reactive chemical species such as superoxide radical, hydroxyl radical, hydrogen peroxide, perosynitrite, or alkyl radicals. These radicals could cause cellular injury and cellular dysfunction by destruction and alteration of lipids, lipoproteins, enzymes, nucleic acids and other cellular biochemicals. Catechins can act as scavengers of free radicals caused by reactive oxygen species and prevent radical damage.

Moreover, green tea catechins can modulate the action of androgens. In many target organs of androgens, such as skin and prostate, testosterone is converted to a more active androgen 5-alpha-dihydrotestosterone (DHT). DHT binds to the androgen receptor and modulates gene expression (Hipakka and Liao, 1998). Actually, the derivatives of catechins such as EGCG and ECG are more active against isozyme I than isozyme II. The inhibitory effect of EGCG is observed when either testosterone or DHT is topically applied, indicating that EGCG activity may be dependent on inhibition of 5-alpha-reductase as well as other mechanisms. Furthermore, sebum production from the male human forehead is also inhibited by topical application of gamma-linolenic acid or EGCG directly to the forehead (Liao, Kao, and Hipakka, 2001). In addition, catechins are also reported to have antiinflammatory effects, and anti-aging effects. Catechins and their derivatives are proved to be effective for certain inflammatory skin diseases such as seborrheic dermatitis, rosacea, and psoriasis.

However, catechins and their derivatives are easily oxidized. Hence, even though catechins and their derivatives can be used for many medical applications, the medical compositions of catechins, especially those for dermatological use cannot be stored for a long time. In other words, since the green tea polyphenols (such as catechins or derivatives thereof) are easily oxidized upon exposure to air, stable topical compositions or mixtures of these polyphenols are not available.

There is thus a general need for a stabilized pharmaceutical and cosmetic composition of catechins or derivatives thereof.

### BRIEF SUMMARY OF THE INVENTION

Accordingly, an embodiment of the present invention is directed to a stabilized pharmaceutical and cosmetic composition of catechins or derivatives thereof, or a method for stabilizing the composition of catechins or derivatives thereof. The pharmaceutical composition disclosed here can inhibit the oxidation of the catechins and derivatives thereof, and therefore extend the shelf life of the pharmaceutical composition of catechins and increase the medical application of the compositions.
To achieve these and other advantages, and in accordance with the purpose of the present invention as embodied and broadly described, there is provided a stabilized pharmaceutical and cosmetic composition of catechins or derivatives thereof. The stabilized pharmaceutical and cosmetic composition of the present invention comprises: 0.01 wt% to 20 wt% antioxidant based on the total weight of the composition; 0.01 wt% to 20 wt% metal chelating agent based on the total weight of the composition; 0.05 wt% to 5 wt% catechins or derivatives thereof based on the total weight of the composition; and water.

Also in accordance with the present invention, there is provided a method for stabilizing the pharmaceutical composition of catechins or derivatives thereof. The method of eth present invention comprises the step of mixing 0.01 wt% to 20 wt% antioxidant; 0.01 wt% to 20 wt% metal chelating agent; 0.05 wt% to 5 wt% catechins or derivatives thereof; and water together. The weight percentages of the components are all based on the total weight of the composition.

The catechins or derivatives here can be any conventional catechins and related derivatives. Preferably, the catechins or the related derivatives are (-)-catechins, (+)-catechins, (-)-epicatechins (EC), (-)-epigallocatechins (EGC), (-)-epicatechin-3-gallates (ECG), (-)-gallocatechin-3-gallates (GCG), (-)-epigallocatechin-3-gallates (EGCG), or the combination thereof. More preferably, the catechins or the derivatives are (-)-catechins, (+)-catechins, (-)-epicatechins (EC), (-)-epigallocatechins (EGC), (-)-epicatechin-3-gallates (ECG), (-)-gallocatechin-3-gallates (GCG), (-)-epigallocatechin-3-gallates (EGCG), or the combination thereof extracted from the tea extracts. The composition of the present invention can selectively further comprising specific components for enhancing the medical effects of the pharmaceutical effects. Preferably, gallates, derivatives of gallates, UV-absorbing reagent for blocking UV light, or the combination thereof are added.

The antioxidants of the composition of the present invention can be any conventional antioxidants used in cosmetics composition, or conventional medical compositions. Of course, more than one antioxidant is generally used, too. According to the invention, favorable antioxidants that can be used are any antioxidants suitable or customary for cosmetic compositions, or conventional medical compositions. Preferably, the antioxidants of the compositions of the present invention are selected from the group consisting of aminoacids derivatives thereof, imidazoles and derivatives thereof, peptides, chlorogenic and derivatives thereof, lipoic and derivatives thereof, aurothioglucose, propylthioruacil and thiols, folic acid or derivatives thereof, ubiquinone and ubiquinol or derivatives thereof, vitamin C and derivatives, tocopherols and derivatives, vitamin A and derivatives and coniferyl benzoate of benzoin, rutinic acid and derivatives thereof, .alpha.-glycosylrutin, ferulic acid, furfurylideneglucitol, carnosine, butylhydroxytoluene, butylhydroxyanisole, nordihydroguaiacic acid, nordihydroguaiaretic acid, trihydroxybutyrophenone, uric acid and derivatives thereof, mannose and derivatives thereof, zinc and derivatives thereof, selenium and derivatives thereof, stilbenes and derivatives thereof, and the derivatives (salts, esters, ethers, sugars, nucleotides, nucleosides, peptides and lipids) of said active substances which are suitable according to the invention. The thiols illustrated above can be any conventional thiols functioning as antioxidants. Preferably, the thiol is selected from the group consisting of thioredoxin, glutathione, cysteine, cystine, cystamine, sulfur, oxygenated sulfur acid, sulfate, sulfite, meta-bisulfite, thiosulfate, dilaurylthiodipropionate, disteary thiodipropionate, thiodipropionic acid, thionine sulfones, pentaand sulfoximine compound, and derivatives thereof. The sulfoximine compounds can be any conventional sulfoximine for being antioxidants. Preferably, the sulfoximine is buthionine sulfoximines, homocysteine sulfoximine, buthionine sulfones, penta- heptathioninesulfoximines, hexa-heptathioninesulfoximines, or the combination thereof. The amino acid illustrated above can be any conventional amino acid for being antioxidants. Preferably, the amino acid of the composition of the present invention is glycine, histidine, tyrosine, tryptophan, or the combination thereof. The lipoic acid illustrated above can be any conventional lipoic acid for being antioxidants. Preferably, the lipoic acid of the composition of the present invention is dihydrolipoic acid. The derivatives of vitamin C illustrated above can be any conventional derivatives of vitamin C for being antioxidants. Preferably, the derivatives of vitamin C of the composition of the present invention are ascorbyl palmitates, Mg ascorbyl phosphates, ascorbyl acetates, or the combination thereof. The derivatives of vitamin E illustrated above can be any conventional derivatives of vitamin E for being antioxidants. Preferably, the derivatives of vitamin E of the composition of the present invention are vitamin E acetate. Likewise, the derivatives of vitamin A illustrated above can be any conventional derivatives of vitamin A for being antioxidants. Preferably, the derivatives of vitamin A of the composition of the present invention are vitamin A palmitate. The derivatives of zinc illustrated above can be any conventional derivatives of zinc for being antioxidants. Preferably, the derivative of zinc of the composition of the present invention is ZnO, ZnSO₄, or the combination thereof. The derivatives of selenium illustrated above can be any conventional derivatives of selenium for being antioxidants. Preferably, the derivatives of selenium of the composition of the present invention are selenomethionines. The derivatives of stilbenes illustrated above can be any conventional derivatives of stilbenes for being antioxidants. Preferably, the derivatives of stilbenes of the composition of the present invention are stilbene oxide, and more preferably, trans-stilbene oxide.

The amount of the abovementioned antioxidants (one or more compounds) in the composition of the present invention is preferably from 0.01 to 20% by weight, particularly preferable from 0.05 to 10% by weight based on the total weight of the composition of the present invention. If vitamin E and /or derivatives thereof are used as the antioxidants, their respective concentrations are advantageously chosen from the range of 0.01-10% by weight, based on the total weight of the composition of the present invention. Likewise, if vitamin A and /or derivatives thereof are used as the antioxidants, their respective concentrations are advantageously chosen from the range of 0.01-10% by weight, based on the total weight of the composition of the present invention.

The metal chelating agents of the composition of the present invention can be any conventional metal chelating agent used in cosmetics composition, or conventional medical compositions. Of course, more than one metal chelating agent is generally used, too. According to the invention, favorable metal chelating agents which can be used are any metal chelating agents suitable or customary for cosmetic compositions, or conventional medical compositions. Preferably, the metal chelating agents of the compositions of the present invention are selected from the group consisting of alpha.-hydroxy fatty acids, palmitic acid, phytic acid, lactofreein, .alpha.-hydroxy acids, humic acic, bile acid, bile extracts, EDTA, EGTA and derivatives thereof, unsaturated fatty acids and derivatives thereof, vitamin C and derivatives, tocopherols and derivatives, vitamin A and derivatives. The alpha.-hydroxy fatty acids illustrated above can be any conventional alpha.-hydroxy fatty acids for being antioxidants. Preferably, the alpha.-hydroxy fatty acid of the composition of the present invention is citric acid, lactic acid, malic acid, or the combination thereof.

The composition of the present invention can further comprises cosmetic auxiliaries such as those conventionally used in such preparations, e.g., preservatives, perfumes antifoams which have a coloring effect, thickeners, moisturizers and /or humectants, fats, oils, waxes or other conventional constituents of a cosmetic or dermatological formulation, such as alcohols, polyols, polymers, foam stabilizers organic solvents or silicon oils.

The oils illustrated above can be any conventional oils for being lipid phase. Preferably, the oils of the composition of the present invention are mineral oils, mineral waxes, triglycerides of capric acid, triglycerides of caprylic acid, or the combination thereof. More preferably, the oils can be branched and unbranched hydrocarbons, hydrocarbon waxes, dialkyl ethers, saturated alcohols, unsaturated alcohols, branched alcohols, unbranched alcohols, polysorbate and also fatty acid trigylcerides, namely the triglycerol esters of saturated and /or unsaturated, branched and/or unbranched alkanecarboxylic acids of chain length from 8 to 24. Most preferably the oils of the composition of the present invention are castor oil. The fats illustrated above can be any conventional natural wax or synthetic fatty wax for being lipid phase. Preferably, the fats used in the composition of the present invention are esters of fatty acids with alcohols of low carbon number. More preferably, the fats of the composition of the present invention are esters of fatty acids with isorpopanol, propylene glycol, or glycerol.

The silicon oils illustrated above can be any conventional silicon oils for being lipid phase or thickeners. Preferably, the silicon oils of the composition of the present invention are dimethylpolysiloxanes, diethylpolysiloxanes, diphenyl-polysiloxanes, cyclmethicone (octamethylcyclotetrasiloxane), hexamethylcyclotrisiloxane, polydimethylsiloxane, poly(methylphenylsiloxane), or the combination thereof.

The alcohols illustrated above can be any conventional oils for being auxiliaries. Preferably, the alcohols are monoalcohols, diols or polyols of low carbon number. More preferably, the alcohol is isopropanol, propylene glycol, glycerol, ethylene glycol, ethylene glycol monoethyl or monobutyl ether, propylene glycol monomethyl, monoethyl or monobutyl ether, diethylene glycol monomethyl, monoethyl ether and analogous products.

The thickeners illustrated above can be any conventional thickeners for being auxiliaries. Preferably, the alcohols are monoalcohols, diols or polyols of low carbon numbe. More preferably, the thickener is silicon dioxide, dodecylsulfate, and sodium salt of dodecylsulfate, aluminum silicates, hyaluronic acid, xanthan gum, polysaccharides and the combination thereof.

For emulsifying the compositions of the present invention, olegels, or surfactants for emulsion can be selectively added to the composition. Preferably, the olegels are the group of esters of saturated and /or unsaturated, branched and/or unbranched alkanecarboxylic acids having a chain length of from 3 to 30 carbon atoms and saturated and/or unsaturated, branched and/or unbranched alcohols having a chain length of from 3 to 30 carbon atoms, or the esters of aromatic carboxylic acids and saturated and/or unsaturated, branched and/or unbranched alcohols having a chain length of from 3 to 30 carbon atoms. Preferably, the olegel is selected from the group consisting of isopropyl myristate, isopropyl palmitate, isopropyl stearate, isopropyl oleate, n-butyl stearate, n-hexyl laurate, n-ethylhexyl laurate, 2-hexyldecyl stearate, 2-octyldodecyl palmitate, oleyl oleate, oleyl erucate, erucyl oleate, erucyl erucate and esters of jojoba oil.

For gel preparation, the total amount of alcohol is in the range of 30-85% by weight based on total weight of the compositions. For cosmetic cream preparation, the total amount of alcohol is advantageously chosen from 10-50% by weight base on total weight of the compositions. The amount of EGCG ranges from 0.01 % to 20% based on the total weight of the composition.

The pH of the composition of the present invention is carefully controlled for stabilizing the catechins or derivatives thereof. Generally, the pH is controlled below 7.4. Preferably, the pH of the composition of the present invention is controlled in a range from 1.8 to 6.4.

The composition of the present invention can be used for any conventional medical purposes. Preferably, the composition of the present invention is used for treating skin diseases or skin disorders. More preferably, the composition of the present invention is used for treating acne, seborrheic dematitis, rosacea, psoriasis, androgenetic alopecia (male pattern baldness), hirsutism, actinic keratosis, and skin cancer.

Additional features and advantages of the present invention will be set forth in part in the description which follows, and in part will be obvious from the description, or may be learned by practice of the present invention. The features and advantages of the present invention will be realized and attained by means of the elements and combinations particularly pointed out in the henceforth-appended claims.

It is to be understood that both the foregoing general description and the following detailed description are exemplary and explanatory only and are not restrictive of the present invention, as claimed.

### DESCRIPTION OF THE EMBODIMENTS

Reference will now be made in detail to present embodiments of the present invention, examples of which are illustrated in the accompanying drawings. Wherever possible, the same reference numbers will be used throughout the drawings to refer to the same or like parts.

Since EGCG is most easily oxidized, in many studies we used EGCG as example. Initial approach was to determine if EGCG could be incorporated and remained stable in commercially available lotions or creams. EGCG was dissolved in ethanol and was added to lotion, cream, or ointment. These were either in skin care products or prescription medicines, in over 100 variations. These EGCG preparations were homogeneous and were stored at room temperature. However, all of them changed to brownish color within days or weeks. The brownish color was the result of oxidation of EGCG. It becomes obvious that stabilization of EGCG is essential.

The examples below serve to illustrate the present invention without limiting it. Unless stated otherwise, all amounts, proportions and percentages are based on the weight and the total amount or on the total weight of the preparations. The general procedure is, the organic reagents were mixed and was stirred at a temperature between 65-70 degree Celsius (C) for 30 min. and separately, aqueous phase was mixed and this then added to the organic phase at a temperature between 65-70 degree Celsius (C) and stirred for additional 15 min. EGCG (98%) was then added at this temperature and further stirred for 15 min.

Furthermore, for following analysis, the EGCG is extracted from the compositions by the extraction of 100mg of said formulation with 2 mL of distilled water with constant shaking. Filter an aliquot of the solution directly into an HPLC-vial using a membrane filter and inject 20 micro liter into the HPLC system. Integration, calibration and calculation are automatically performed with the software and the retention time of EGCG and its calibration is completed by the co-injection of standard 1% EGCG aqueous solution for each HPLC analysis.

Comparative example 1 Preparation of EGCG Skin Cream

| **Aqueous Phase:** | |
|---|---|
| Xanthan Gum | 0.12g |
| Dist. Water | 26.4 mL |

| **Organic phase:** | |
|---|---|
| 1-hexadecanol | 1.2g |
| 1-octadecanol | 0.4g |
| sorbitan monostearate | 1.2g |
| polyethylene glycol distearate | 0.8g |
| palmitic acid | 0.6g |
| dodecyl sulfate, sodium salt(SDS) | 0.12g |
| cyclomethicone | 3.2 mL |
| dimethicone-350 | 1.38 mL |
| glycerol | 3.2 mL |
| polysorbate-20 | 1.38 mL |

The aqueous phase was heated at 70 degree C with constant stirring. Homogenizer was used to enhance the dissolution of xanthan gum and it was heated for an hour. It was cooled to room temperature and after 24 hr. at room temperature it was again heated to 70 degree C for another hour with stirring.

In addition, 0.2g of 98% EGCG was added along with 0.08g of sodium thiosulfate and it was then heated and stirred for 15 min. at this temperature.

The organic phase was combined and heated for 1 hr at 70 degree C. Aqueous phase was then poured into the organic phase and was homogenized for 15 minutes at this temperature. Moreover, there is no citric acid in the composition of the present comparative example.

The components illustrated above are mixed by the steps illustrated in the former paragraph. The composition prepared is laid in a chamber at room temperature for one week. The composition is then analyzed by HPLC for identifying the retained amount of the EGCG. The HPLC (high pressure liquid chromatography) with UV (274 nm) detectors was used for the analysis for EGCG content in the formulation (Lee et.al., 2000, Liao 2001).

The resulted data of HPLC shows that only 13.3 area percent of EGCG is left one week later.

Example 1

The composition is prepared and analyzed through the same steps illustrated in comparative example 1 except the components and the weight percentages of each component are replaced by the combination listed below:

| | By weight (%) |
|---|---|
| Stearic acid | 2.7 |
| Palmitic acid | 2.7 |
| 1-hexadecanol | 5.4 |
| 1-octadecanol | 2.7 |
| sortitan monostearate | 3.1 |
| propylene glycol | 18 |
| glycerol | 9 |
| triton x-100 | 18 |
| distilled water | 37.5 |
| citric acid | 0.4 |
| EGCG | 0.5 |

The resulted data of HPLC shows that most EGCG (more than 78 area peercent) is left without change one week later.

Example 2

The composition is prepared and analyzed through the same steps illustrated in comparative example 1 except the components and the weight percentages of each component are replaced by the combination listed below:

| | By weight (%) |
|---|---|
| Stearic acid | 2.6 |
| Palmitic acid | 2.6 |
| 1-hexadecanol | 5.2 |
| 1-octadecanol | 2.6 |
| sorbitan monostearate | 0.3 |
| propylene glycol | 17.7 |
| glycerol | 8.85 |
| tritonx-100 | 17.7 |
| distilled water | 38.5 |
| citric acid | 0.44 |
| EGCG | 0.5 |
| PEG-40 | 3 |

The resulted data of HPLC shows that most EGCG is left without change one week later.

Example 3

The composition is prepared and analyzed through the same steps illustrated in comparative example 1 except the components and the weight percentages of each component are replaced by the combination listed below:

| | By weight (%) |
|---|---|
| 1-hexadecanol | 5.95 |
| 1-octadecanol | 2 |
| sorbitan monostearate | 4 |
| polyethylene glycol distearate | 4 |
| palmitic acid | 2 |
| dodecyl sulfate, sodium salt | 0.4 |
| citric acid | 0.2 |
| cyclomethicone | 10 |
| polysorbate-20 | 4 |
| glycerol | 10 |
| distilled water | 53.2 |
| EGCG | 0.5 |

The resulted data of HPLC shows that most EGCG is left without change one week later.

Example 4

The composition is prepared and analyzed through the same steps illustrated in comparative example 1 except the components and the weight percentages of each component are replaced by the combination listed below:

| | By weight (%) |
|---|---|
| Methyl 4-hydroxybenzoate | 0.1 |
| 1-hexadecanol | 4 |
| 1-octadecanol | 1.5 |
| sorbitan monostearate | 4 |
| polyethylene glycol distearate | 3 |
| palmitic acid | 1.5 |
| dodecyl sulfate, sodium salt | 0.25 |
| xanthan gum | 0.2 |
| citric acid | 0.1 |
| cyclomethicone | 6.5 |
| dimethicone-350 | 3 |
| polysorbate-20 | 3 |
| glycerol | 6.5 |
| distilled water | 66.3 |
| EGCG | 0.5 |

The resulted data of HPLC shows that most EGCG is left without change one week later.

Example 5

The composition is prepared and analyzed through the same steps illustrated in comparative example 1 except the components and the weight percentages of each component are replaced by the combination listed below:

| | By weight (%) |
|---|---|
| 1-hexadecanol | 6 |
| 1-octadecanol | 2 |
| sorbitan monostearate | 4 |
| polyethylene glycol distearate | 4 |
| palmitic acid | 0.1 |
| dodecyl sulfate, sodium salt | 0.4 |
| cyclomethicone | 10 |
| dimethicone-350 | 4 |
| polysorbate-20 | 4 |
| glycerol | 10 |
| distilled water | 53 |
| sodium thiosulfate | 0.5 |
| quercetin | 0.5 |
| EGCG | 0.5 |

The resulted data of HPLC shows that most EGCG is left without change one week later.

Example 6

The composition is prepared and analyzed through the same steps illustrated in comparative example 1 except the components and the weight percentages of each component are replaced by the combination listed below:

| | By weight (%) |
|---|---|
| 1-hexadecanol | 3.5 |
| palmitic acid | 2.3 |
| 1-octadecanol | 1.2 |
| stearic acid | 0.12 |
| cyclomethicone | 14 |
| glycerol | 5.8 |
| dodecyl sulfate, sodium salt | 2 |
| citric acid | 1 |
| sorbitol | 2 |
| carbomer | 0.5 |
| avobenzene | 1 |
| octocrylene | 3.3 |
| distilled water | 62 |
| EGCG | 0.5 |

The resulted data of HPLC shows that most EGCG is left without change one week later.

Example 7

The composition is prepared and analyzed through the same steps illustrated in comparative example 1 except the components and the weight percentages of each component are replaced by the combination listed below:

| | By weight (%) |
|---|---|
| Sesame oil | 10.9 |
| Ethylene glycol methyl ether | 2.2 |
| Triethanol amine | 2.2 |
| Aluminum stearate | 0.3 |
| Ascorbic acid-6-palmitate | 0.3 |
| Isopropylpalmitate | 2.2 |
| Stearic acid | 0.8 |
| 1-octadecanol | 1.3 |
| carbomer | 0.28 |
| ethylene glycol phenyl ether | 2.2 |
| cyclomethicone | 10.9 |
| dodecyl sulfate, sodium salt | 0.22 |
| triton x-100 | 8 |
| polyethylene glycol distearate | 2.2 |
| distilled water | 56 |

The resulted data of HPLC shows that most EGCG is left without change one week later.

Example 8

The composition is prepared and analyzed through the same steps illustrated in comparative example 1 except the components and the weight percentages of each component are replaced by the combination listed below:

| | By weight (%) |
|---|---|
| Citric acid | 0.1 |
| Methyl 4-hydroxybenzoate | 0.1 |
| 1-hexadecanol | 4 |
| 1-octadecanol | 1.3 |
| sorbitan monostearate | 2.5 |
| PEG-40 hydrogenated caster oil | 2 |
| Polyethylene glycol distearate | 2.5 |
| Palmitic acid | 1.5 |
| Dodecyl sulfate, sodium salt | 0.25 |
| Xanthan gum | 0.2 |
| Propylene glycol | 5 |
| Dimethicone-350 | 5 |
| Polysorbate-20 | 3 |
| Glycerol | 7 |
| Distilled water | 65.5 |
| EGCG | 0.5 |

The resulted data of HPLC shows that most EGCG is left without change one week later.

Example 9

The composition is prepared and analyzed through the same steps illustrated in comparative example 1 except the components and the weight percentages of each component are replaced by the combination listed below:

| | |
|---|---|
| Citric acid | 0.1 |
| Sodium thiosulfate | 0.18 |
| Methyl 4-hydroxybenzoate | 0.1 |
| 1-hexadecanol | 3.1 |
| 1-octadecanol | 1.5 |
| sosrbitan monostearate | 2.6 |
| polyethylene glycol distearate | 2.6 |
| palmitic acid | 1 |
| dodecyl sulfate sodium salt | 0.25 |
| xanthan gum | 0.2 |
| cyclomethicone | 1.15 |
| dimethicone-350 | 1.15 |
| polysorbate-20 | 2.6 |
| glycerol | 6.7 |
| distilled water | 75.7 |
| EGCG | 0.5 |

The resulted data of HPLC shows that most EGCG is left without change one week later.

Example 10

The composition is prepared and analyzed through the same steps illustrated in comparative example 1 except the components and the weight percentages of each component are replaced by the combination listed below:

| | By weight (%) |
|---|---|
| Stearic acid | 2.7 |
| Palmitic acid | 2.7 |
| 1-hexadecanol | 5.4 |
| 1-octadecanol | 2.7 |
| sorbitan monostearate | 3.1 |
| propylene glycol | 18 |
| glycerol | 9 |
| triton X-1 00 | 18 |
| distilled water | 38 |
| citric acid | 0.4 |
| EGCG | 0.5 |
| Carbomer | 0.5 |

The resulted data of HPLC shows that most EGCG is left without change one week later.

Example 11

The composition is prepared and analyzed through the same steps illustrated in comparative example 1 except the components and the weight percentages of each component are replaced by the combination listed below:

| | By weight (%) |
|---|---|
| Dodecyl sulfate, sodium salt | 5.3 |
| Citric acid | 0.1 |
| Methyl 4-hydroxybenzoate | 0.1 |
| 1-hexadecanol | 5.3 |
| sorbitan monostearate | 3.2 |
| polyethylene glycol distearate | 2.1 |
| sodium thiosulfate | 0.1 |
| PEG-40 | 5.2 |
| Glycolic acid | 0.5 |
| Glycerol | 1.6 |
| Distilled water | 55.5 |
| Cocamidopropylbetaine | 8.5 |
| EGCG | 0.1 |

The resulted data of HPLC shows that most EGCG is left without change one week later.

Example 12

The composition is prepared and analyzed through the same steps illustrated in comparative example 1 except the components and the weight percentages of each component are replaced by the combination listed below:

| | By weight (%) |
|---|---|
| Dodecyl sulfate sodium salt | 3.8 |
| Citric acid | 0.5 |
| Methyl 4-hydroxybenzoate | 0.1 |
| Propyl 4-hydroxybenzoate | 0.1 |
| 1-hexadecanol | 3.8 |
| palmitic acid | 0.77 |
| sorbitan monostearate | 2.3 |
| polyethylene glycol distearate | 1.5 |
| PEG-40 | 2.3 |
| Glycolic acid | 0.5 |
| Propylene glycol | 3.8 |
| Glycerol | 11.57 |
| Distilled water | 62.5 |
| Cocamidopropylbetaine | 6.2 |
| EGCG | 0.05 |

The resulted data of HPLC shows that most EGCG is left without change one week later.

Example 13

The composition is prepared and analyzed through the same steps illustrated in comparative example 1 except the components and the weight percentages of each component are replaced by the combination listed below:

| | By weight (%) |
|---|---|
| Citric acid | 1 |
| Methyl 4-hydroxybenzoate | 1 |
| 1-hexadecanol | 4 |
| 1-octadecanol | 1 |
| sorbitan monostearate | 3 |
| polyethylene glycol distearate | 3 |
| pamitic acid | 1 |
| dodecyl sulfate, sodium salt | 0.5 |
| xanthan gum | 0.2 |
| cyclomethicone | 5 |
| dimethicone-350 | 3 |
| polysorbate-20 | 3 |
| glycerol | 6 |
| distilled water | 70.1 |
| EGCG | 5 |

The resulted data of HPLC shows that most EGCG is left without change one week later.

Example 14

The composition is prepared and analyzed through the same steps illustrated in comparative example 1 except the components and the weight percentages of each component are replaced by the combination listed below:

| | By weight (%) |
|---|---|
| Citric aicd | 0.1 |
| Sodium thiosulfate | 0.2 |
| Methyl 4-hydroxybenzoate | 0.1 |
| 1-hexadecanol | 3.3 |
| 1-octadecanol | 1.3 |
| sorbitan monostearate | 2.6 |
| polyethylene glycol distearate | 2.6 |
| palmitic acid | 1.3 |
| dodecyl sulfate, sodium salt | 0.5 |
| xanthan gum | 0.15 |
| cyclomethicone | 4 |
| dimethicone-350 | 2.6 |
| polysorbate-20 | 2.6 |
| glycerol | 6.5 |
| distilled water | 68.3 |
| Triton X-100 | 2 |
| EGCG | 2 |

The resulted data of HPLC shows that most EGCG is left without change one week later.

Example 15

The composition is prepared and analyzed through the same steps illustrated in comparative example 1 except the components and the weight percentages of each component are replaced by the combination listed below:

| | By weight (%) |
|---|---|
| Citric acid | 0.3 |
| Xanthan gum | 0.15 |
| Cyclomethicone | 2.4 |
| Glycerol | 6.5 |
| Dimethicone-350 | 5 |
| Propylene glycol | 6.5 |
| 1-hexadecanol | 3.5 |
| palmitic acid | 1 |
| L-ascorbic acid-6-palmitate | 0.5 |
| Sorbitan monostearate | 3 |
| Dodecyl sulfate, sodium salt | 0.5 |
| Magnesium sulfate | 0.2 |
| EGCG | 0.5 |
| Distilled water | 70 |

The resulted data of HPLC shows that most EGCG is left without change one week later.

Example 16

The composition is prepared and analyzed through the same steps illustrated in comparative example 1 except the components and the weight percentages of each component are replaced by the combination listed below:

| | By weight (%) |
|---|---|
| Citric acid | 0.3 |
| Xanthan gum | 0.15 |
| Cyclomethicone | 2.4 |
| Glycerol | 6.5 |
| Dimethicone-350 | 5 |
| Propylene glycol | 6.5 |
| 1-hexadecanol | 3.5 |
| palmitic acid | 1 |
| L-ascorbic acid-6-palmitate | 0.5 |
| Sorbitan monostearate | 3 |
| Dodecyl sulfate, sodium salt | 0.5 |
| sodium hydrogen sulfate | 0.2 |
| EGCG | 0.5 |
| Distilled water | 70 |

The resulted data of HPLC shows that most EGCG is left without change one week later.

Example 17

The composition is prepared and analyzed through the same steps illustrated in comparative example 1 except the components and the weight percentages of each component are replaced by the combination listed below:

| | By weight (%) |
|---|---|
| Citric acid | 0.3 |
| Xanthan gum | 0.15 |
| Cyclomethicone | 2.4 |
| Glycerol | 6.5 |
| Dimethicone-350 | 5 |
| Propylene glycol | 6.5 |
| 1-hexadecanol | 3.5 |
| palmitic acid | 1 |
| L-ascorbic acid-6-palmitate | 0.5 |
| Sorbitan monostearate | 3 |
| Dodecyl sulfate, sodium salt | 0.5 |
| sodium meta bisulfite | 0.2 |
| EGCG | 0.5 |
| Distilled water | 70 |

The resulted data of HPLC shows that most EGCG is left without change one week later.

Example 18

The composition is prepared and analyzed through the same steps illustrated in comparative example 1 except the components and the weight percentages of each component are replaced by the combination listed below:

| | By weight (%) |
|---|---|
| Citric acid | 0.3 |
| Xanthan gum | 0.15 |
| Cyclomethicone | 2.4 |
| Glycerol | 6.5 |
| Dimethicone-350 | 5 |
| Propylene glycol | 6.5 |
| 1-hexadecanol | 3.5 |
| palmitic acid | 1 |
| L-ascorbic acid-6-palmitate | 0.5 |
| Sorbitan monostearate | 3 |
| Dodecyl sulfate, sodium salt | 0.5 |
| sodium meta bisulfite | 0.2 |
| EGCG | 0.5 |
| Distilled water | 70 |

The resulted data of HPLC shows that most EGCG is left without change one week later.

Example 19

The composition is prepared and analyzed through the same steps illustrated in comparative example 1 except the components and the weight percentages of each component are replaced by the combination listed below:

| | By weight (%) |
|---|---|
| Citric acid | 0.3 |
| Xanthan gum | 0.15 |
| Cyclomethicone | 2.4 |
| Glycerol | 6.5 |
| Dimethicone-350 | 5 |
| Propylene glycol | 6.5 |
| 1-hexadecanol | 3.5 |
| palmitic acid | 1 |
| L-ascorbic acid-6-palmitate | 0.5 |
| Sorbitan monostearate | 3 |
| Dodecyl sulfate, sodium salt | 0.5 |
| sodium thiosulfate | 0.2 |
| EGCG | 0.5 |
| Distilled water | 70 |

The resulted data of HPLC shows that most EGCG is left without change one week later.

Example 20

The composition is prepared and analyzed through the same steps illustrated in comparative example 1 except the components and the weight percentages of each component are replaced by the combination listed below:

| | By weight (%) |
|---|---|
| Citric acid | 0.3 |
| Xanthan gum | 0.15 |
| Cyclomethicone | 2.4 |
| Glycerol | 6.5 |
| Dimethicone-350 | 5 |
| Propylene glycol | 6.5 |
| 1-hexadecanol | 3.5 |
| palmitic acid | 1 |
| L-ascorbic acid-6-palmitate | 0.5 |
| Sorbitan monostearate | 3 |
| Dodecyl sulfate, sodium salt | 0.5 |
| sodium bisulfite | 0.2 |
| EGCG | 0.5 |
| Distilled water | 70 |

The resulted data of HPLC shows that most EGCG is left without change one week later.

Example 21 (Therapeutic gel with EGCG)

The composition is prepared and analyzed through the same steps illustrated in comparative example 1 except the components and the weight percentages of each component are replaced by the combination listed below:

| | By weight (%) |
|---|---|
| Glycerol | 10-20 |
| Propylene glycol | 0-20 |
| Butylated hydroxytoluene | 0-0.1 |
| Ascorbic aicd | 0-0.2 |
| Sodium thiosulfate | 0-3 |
| EDTA | 0-0.5 |
| Citrate buffer (10 mM, pH 3.0) | 25-40 |
| Distilled water | 10-50 |
| Methyl paraben | 0.2 |
| Propyl paraben | 0.04 |
| Isopropyl alcohol | 10-50 |
| HPC-HF | 0-2 |
| Total | 100 |

The resulted data of HPLC shows that most EGCG is left without change one week later.

.The result illustrated above shows that the composition of the present invention can effectively stabilize the catechins or their derivatives.

Other embodiments of the present invention will be apparent to those skilled in the art from consideration of the specification and practice of the present invention disclosed herein. It is intended that the specification and examples be considered as exemplary only, with a true scope and spirit of the present invention being indicated by the following claims.

## Claims

1. A stabilized pharmaceutical and cosmetic composition of catechins or derivatives thereof, comprising:
0.01 wt% to 20 wt% antioxidants based on the total weight of the composition;
0.01 wt% to 20 wt% metal chelating agents based on the total weight of the composition;
0.05 wt% to 5 wt% catechins or derivatives thereof based on the total weight of the composition; and
water.

2. The stabilized pharmaceutical and cosmetic composition of claim 1, wherein the catechin or the derivative is (-)-catechin, (+)-catechin, (-)-epicatechin(EC), (-)-epigallocatechin(EGC), (-)-epicatechin-3-gallate (ECG), (-)-gallocatechin-3-gallate (GCG), (-)-epigallocatechin-3-gallate (EGCG), or the combination thereof.

3. The stabilized pharmaceutical and cosmetic composition of claim 1, wherein the antioxidants are selected from the group consisting of aminoacids derivatives thereof, imidazoles and derivatives thereof, peptides, chlorogenic and derivatives thereof, lipoic and derivatives thereof, aurothioglucose, propylthioruacil and thiols.

4. The stabilized pharmaceutical and cosmetic composition of claim 3, wherein at least one thiol is selected from the group consisting of thioredoxin, glutathione, cysteine, cystine, cystamine, sulfur, oxygenated sulfur acid, sulfate, sulfite, meta-bisulfite, thiosulfate, dilaurylthiodipropionate, disteary thiodipropionate, thiodipropionic acid, thionine sulfones, pentaand sulfoximine compound, and derivatives thereof.

5. The stabilized pharmaceutical and cosmetic composition of claim 1, wherein the antioxidants are folic acid or derivatives thereof, ubiquinone and ubiquinol or derivatives thereof, vitamin C and derivatives, tocopherols and derivatives, vitamin A and derivatives and coniferyl benzoate of benzoin, rutinic acid and derivatives thereof, .alpha.-glycosylrutin, ferulic acid, furfurylideneglucitol, carnosine, butylhydroxytoluene, butylhydroxyanisole, nordihydroguaiacic acid, nordihydroguaiaretic acid, trihydroxybutyrophenone, uric acid and derivatives thereof, mannose and derivatives thereof, zinc and derivatives thereof, selenium and derivatives thereof, stilbenes and derivatives thereof.

6. The stabilized pharmaceutical and cosmetic composition of claim 5, wherein the antioxidant is ascorbyl palmitate, Mg ascorbyl phosphate, ascorbyl acetate, vitamin E acetate, vitamin A palmitate, ZnO, ZnSO₄, selenomethionine, stilbene oxide, or the combination thereof.

7. The stabilized pharmaceutical and cosmetic composition of claim 1, wherein the metal chelating agent is selected from the group consisting of alpha.-hydroxy fatty acids, palmitic acid, phytic acid, lactofreein, .alpha.-hydroxy acids, humic acic, bile acid , bile extracts, EDTA, EGTA and derivatives thereof, unsaturated fatty acids and derivatives thereof, vitamin C and derivatives, tocopherols and derivatives, vitamin A and derivatives.

8. The stabilized pharmaceutical and cosmetic composition of claim 7, wherein the .alpha.-hydroxy acid is citric acid, lactic acid, malic acid, or the combination thereof.

9. The stabilized pharmaceutical and cosmetic composition of claim 1, further comprising mineral oils, mineral waxes, fat waxes, silicone oils, oleogels, surfactants for emulsion, and the combination thereof.

10. The stabilized pharmaceutical and cosmetic composition of claim 1, further comprising gallates or derivatives of gallates.

11. The stabilized pharmaceutical and cosmetic composition of claim 9, wherein the surfactant is aliphatic unsaturated fatty acids.

12. The stabilized pharmaceutical and cosmetic composition of claim 1, further comprising an UV-absorbing reagent for blocking UV light.

13. The stabilized pharmaceutical and cosmetic composition of claim 1, wherein the catechins or derivatives thereof are extracted from the tea extracts.

14. The stabilized pharmaceutical and cosmetic composition of claim 1, which is used for treating skin diseases.

15. The stabilized pharmaceutical and cosmetic composition of claim 1, wherein the pH is from 1.8 to 6.4.
